# EUROPÄISCHE PATENTSCHRIFT

(11) **EP 3 669 621 B1**
(45) Veröffentlichungstag und Bekanntmachung des Hinweises auf die Patenterteilung: **29.05.2024**
(21) Anmeldenummer: 18759051.8
(22) Anmeldetag: 08.08.2018
(51) Int. Cl.: H05H 1/24, A61L 2/14

(54) **PLASMAGENERATORMODUL UND DESSEN VERWENDUNG**
PLASMA GENERATOR MODULE AND USE THEREOF
MODULE DE GÉNÉRATION DE PLASMA ET UTILISATION DUDIT MODULE

(30) Priorität: 16.08.2017 DE 102017118652
(43) Veröffentlichungstag der Anmeldung: 24.06.2020
(73) Patentinhaber: DBD Plasma GmbH, 37085 Göttingen (DE)
(72) Erfinder: VIÖL, Wolfgang, 37139 Adelebsen (DE); WIENEKE, Stephan, 37079 Göttingen (DE); GREDNER, Alexander, 37133 Friedland (DE); FREIER, Daniel, 37120 Bovenden (DE)
(74) Vertreter: REHBERG HÜPPE + PARTNER
(86) Internationale Anmeldenummer: PCT/EP2018/071517
(87) Internationale Veröffentlichungsnummer: WO 2019/034496

(56) Entgegenhaltungen:
- EP-A2- 1 767 068
- DE-A1-102005 029 360
- JP-A- 2003 338 399
- KR-B1- 101 709 167
- US-A1- 2008 061 035

## Beschreibung

### TECHNISCHES GEBIET DER ERFINDUNG

Die Erfindung bezieht sich auf ein Modularray aus mehreren Plasmageneratormodulen zum Generieren eines physikalischen Plasmas durch dielektrische behinderte Entladung bei Atmosphärendruck. Weiterhin bezieht sich die Erfindung auf eine Verwendung eines eines solchen Modularrays.

Spezieller betrifft die Erfindung Plasmageneratormodule jeweils mit zwei linear langgestreckten Elektroden, von denen mindestens eine mit einer dielektrischen Abschirmung versehen ist und die in einem seitlichen Elektrodenabstand parallel zueinander verlaufen, mit Anschlusseinrichtungen zum Anschließen der Elektroden an eine Hochspannungsquelle und mit zwei flächigen Gasleitelementen zum Leiten von Arbeitsgas zwischen die Elektroden.

### STAND DER TECHNIK

Aus J. Ehlbeck et al.: Low Temperature Atmospheric Pressure Plasma Sources for Microbial Decontamination, Journal of Physics D: Applied Physics, IOP Publishing, 2011, 44 (1), pp. 13002 (https://hrl.archives-ouvertes.fr/hal-00585169) sind verschiedene Anordnungen von Elektroden bekannt, um durch Anlegen einer Wechselhochspannung in unterschiedlichen Entladungsformen eine dielektrisch behinderte Entladung hervorzurufen, die ein physikalisches Plasma bei Atmosphärendruck generiert. Zu den unterschiedlichen Entladungsformen zählen eine direkte dielektrisch behinderte Entladung zwischen einer Elektrode und einem geerdeten Objekt, wobei die an der Elektrode anliegende Wechselhochspannung gegenüber Erde generiert wird, eine dielektrisch behinderte Entladung zwischen zwei Elektroden, zwischen denen die Wechselhochspannung angelegt wird, wobei das so generierte Plasma als Plasmajet ausgeblasen wird, und eine koplanare Gleitentladung zwischen einer ersten Elektrode und einem Objekt sowie zwischen dem Objekt und einer zweiten Elektrode, wobei die Wechselhochspannung ebenfalls zwischen den Elektroden angelegt wird aber eine direkte dielektrisch behinderte Entladung zwischen den Elektroden zum Beispiel durch eine zusätzliche dazwischen angeordnete Blende aus Dielektrikum verhindert wird.

Je nach Art einer zu beseitigenden mikrobiellen Kontamination beziehungsweise des kontaminierten Objekts kann die eine oder die andere Entladungsform zum Generieren eines dekontaminierenden Plasmas besonders geeignet sein.

Die Behandlung großflächiger Objekte kann es zudem notwendig machen, große Elektroden vorzusehen, um das physikalische Plasma großflächig zu generieren. Dabei erweist es sich jedoch als schwierig, die dielektrisch behinderte Entladung gleichmäßig über alle Bereiche der großen Elektroden hinweg hervorzurufen.

Aus der WO 2014/093513 A1 ist eine Kaltplasma-Vorrichtung zur Keimabtötung auf Lebensmitteloberflächen bekannt, die sowohl eine Elektrodenanordnung für eine Gleitentladung als auch eine Elektrodenanordnung für einen Plasmajet umfasst.

Aus der WO 2011/095245 A1 ist ein Plasmagenerator zur Erzeugung eines Plasmastrahls bekannt, dessen Elektrodenanordnung auch zum Generieren einer direkten dielektrisch behinderten Entladung gegenüber einem zu behandelten Objekt verwendet werden kann.

Die WO 2015/164760 A1 beschreibt eine Behandlung/Entkeimung von fließendem Wasser mittels einer ein physikalisches Plasma generierenden Entladung. Die Entladung unter Ozonbildung wird mit einem Marx-Hochspannungsgenerator hervorgerufen. Dieser umfasst eine Reihe von parallel geschalteten Elektrodenpaaren, um eine Koronaentladung in einer Plasmakammer zu zünden. Dabei ist der Abstand der Elektroden jedes Paars, der etwa 15 bis 40 mm betragen soll, einstellbar.

Die WO 0154464 A1 offenbart einen Dreiphasenplasmagenerator mit einstellbaren Elektroden zum Erzeugen eines heißen Plasmastrahls. Die Elektroden sind einstellbar, um auch bei auftretendem Verschleiß einen Abstand und eine Konfiguration der Elektroden konstant zu halten.

Die WO 2010/083040 A1 offenbart ein Verfahren und eine Vorrichtung zur kontinuierlichen Herstellung von Nano- und Mikropartikeln, zum Beispiel Kolloiden, in Flüssiglösungen. Die Partikel werden in einer Flüssigkeit, zum Beispiel Wasser als aktives Dielektrikum, mit einem einstellbaren Plasma und einer einstellbaren elektrochemischen Verarbeitungstechnik erzeugt. Zur Einstellung des Plasmas ist eine vertikale Höhe von Elektroden gegenüber einer Oberfläche der Flüssigkeit verstellbar.

In der CN 204 598 448 U ist eine fernbedienbare Einstellvorrichtung offenbart, mit der ein Abstand von Elektroden bei einer Vorrichtung zum Generieren eines physikalischen Plasmas durch dielektrisch behinderte Entladung mit fester Entladungsform veränderbar ist.

Aus der US 2008/0061035 A1 ist ein Plasmagenerator bekannt, bei dem der Abstand von Elektroden verstellbar ist, die an Gasführungselementen gelagert, parallel zueinander angeordnet und wechselweise mit den beiden Polen einer Wechselhochspannungsquelle verbunden sind. Zur Verstellung des Abstands werden die Elektroden zusammen mit den Gasführungselementen quer zu ihren Haupterstreckungsrichtungen aufeinander zu bewegt.

Aus der DE 10 2013 113 941 A1 ist ein Gerät zur Erzeugung eines Plasmas und/oder eines angeregten Gases oder Gasgemisches zur Behandlung von Wunden bekannt. Das Gerät umfasst ein Expansionselement, das einen zu behandelnden Wundbereich mit seinem distalen Ende umgibt. Das proximale Ende des Expansionselements ist lösbar mit einem Gehäuse des Geräts an einer Öffnung verbunden, aus der das erzeugte Plasma und/oder angeregte Gas oder Gasgemisch austritt. Als Hochspannungsquelle des bekannten Geräts ist ein in das Gehäuse integrierter piezoelektrischer Transformator vorgesehen. Das kegelstumpfmantelförmige Expansionselement weist Durchbrüche auf. Eine Rückführung mit einer darin integrierten Pumpe führt Gas aus dem Inneren des Expansionselements in den Bereich des Hochspannungsendes des piezoelektrischen Transformators zurück.

Aus der DE 10 2005 029 360 A1 ist ein Verfahren zur kontinuierlichen Plasmabehandlung bei Atmosphärendruck von elektrisch isolierenden Werkstücken bekannt, bei dem ein zu behandelndes Werkstück in einem Abstand unterhalb einer Elektrode angeordnet wird, die aus zwei in Bewegungsrichtung unter Belassung eines Spalts hintereinander angeordneten, sich quer zu der Bewegungsrichtung zumindest für die Breite der zu behandelnden Oberfläche des Werkstücks erstreckenden Barrierenelektroden besteht. Die Elektrode und das Werkstück werden in einer Bewegungsrichtung relativ zueinander in Bewegung versetzt. Dabei wird an die Barrierenelektroden eine Hochspannung in Form einer Wechselspannung angelegt, um zumindest in dem Spalt eine Plasmaentladung zu zünden. Mittels eines Gasstroms wird die Plasmaentladung aus dem Spalt in Richtung der zu behandelnden Oberfläche des Werkstücks getrieben. Die der zu behandelnden Oberfläche des Werkstücks zugewandten Flächen der Barrierenelektroden werden mit der Hochspannung beaufschlagt. Dabei wird eine aufgrund des aus dem Spalts austretenden, in den Bereich zwischen den Barrierenelektroden und der zu behandelnden Oberfläche des Werkstücks strömenden Plasmagases verminderte Zündspannung für eine Zündung einer zwischen den dem Werkstück zugewandten Oberfläche der Barriereelektroden und der zu behandelnden Oberfläche des Werkstücks erreicht und damit ein über die gesamte Breite des Werkstücks wirkendes Plasma gezündet. Im Bereich der Plasmabehandlung entstehende Gase werden abgesaugt. Dazu ist über den Barrierenelektroden ein in Richtung des Werkstücks offenes, tunnelförmiges Gehäuse angeordnet, welches über eine obere Öffnung an eine Absaugung angeschlossen ist.

Aus der US 2008/0061035 A1 ist ein Plasmagenerator mit einer Gasversorgung zur Versorgung mit einem Arbeitsgas und mit einer Mehrzahl von Elektroden zum Erzeugen eines Plasmas unter Verwendung des Arbeitsgases bekannt. Die Elektroden haben die Form langer Stangen, die parallel zueinander in gleichen Abständen angeordnet sind. Die gleichen Abstände zwischen den Elektroden sind einstellbar und die Gasversorgung weist einzelne verstellbar angeordnete Versorgungselemente auf, die so zueinander und zu den Elektroden einstellbar sind, dass sie das Arbeitsgas jeweils zwischen zwei benachbarten Elektroden zuführen.

Aus der WO 2005/125286 A2 ist eine Vorrichtung zur Bearbeitung eines Substrats mittels mehrerer Plasmajets bekannt. Für jedem der Plasmajets umfasst die Vorrichtung ein Behältnis, durch welches ein Trägergas entlang einer Strömungsrichtung hindurchströmt, sowie eine erste Elektrode und eine zweite Elektrode. Die beiden Elektroden sind durch eine dielektrische Barriere voneinander getrennt. Zwischen den Elektroden wird zur Erzeugung eines Plasmas bei Atmosphärendruck eine Wechselspannung angelegt. Die erste Elektrode ist von der zweiten Elektrode bezogen auf die Strömungsrichtung des Trägergases axial und radial beabstandet. Mehrere Behältnisse und zugehörige Paare von Elektroden zur Erzeugung jeweils eines Plasmajets sind in festen Abständen nebeneinander angeordnet, wobei die jeweiligen Strömungsrichtungen des Trägergases parallel zueinander ausgerichtet sind. Die Behältnisse sind dabei an einem gemeinsamen Isoliergehäuse befestigt, in dem ein zu den einzelnen Behältnissen führender Gasverteilungsraum für das Trägergas ausgebildet ist.

### AUFGABE DER ERFINDUNG

Der Erfindung liegt die Aufgabe zugrunde, ein Modularray aus mehreren Plasmageneratormodulen zum Generieren eines physikalischen Plasmas durch dielektrisch behinderte Entladung bei Atmosphärendruck aufzuzeigen, das einen stabilen und sicheren Betrieb ermöglicht, um auch sehr großflächige Objekte einer Plasmabehandlung unterziehen zu können.

### LÖSUNG

Die Aufgabe der Erfindung wird durch ein Modularray aus mehreren Plasmageneratormodulen mit den Merkmalen des unabhängigen Patentanspruchs 1 gelöst. Die abhängigen Patentansprüche 2 bis 11 betreffen bevorzugte Ausführungsformen und die Patentansprüche 12 bis 15 betreffen Verwendungen des erfindungsgemäßen Modularrays.

### BESCHREIBUNG DER ERFINDUNG

Bei einem erfindungsgemäßen Modularray aus mehreren Plasmageneratormodulen zum Generieren eines physikalischen Plasmas durch elektrisch behinderte Entladung bei Atmosphärendruck weisen die Plasmageneratormodule jeweils zwei linear langgestreckte Elektroden, von denen mindestens eine mit einer dielektrischen Abschirmung versehen ist und die in einem seitlichen Elektrodenabstand parallel zueinander verlaufen, Anschlusseinrichtungen zum Anschließen der Elektroden an eine Hochspannungsquelle und zwei flächige Gasleitelementen zum Leiten von Arbeitsgas zwischen die Elektroden auf und grenzt an mindestens eine der beiden Elektroden, auf ihrer der anderen Elektrode abgekehrten Außenseite eine schlitzförmige Mündung eines Absaugkanal an, der sich auf einer Außenseite des an die mindestens eine der beiden Elektroden anschließenden Gasleitelements über dieses Gasleitelement hinweg erstreckt.

Der Absaugkanal schließt über den Bereich vor den Elektroden hinweg an einen von den Gasleitelementen verlängerten Gaszuführkanal an. Der Absaugkanal kann damit genutzt werden, um eine gezielte Durchströmung dieses Bereichs vor den Elektroden, in dem eine Plasmabehandlung mit den Plasmageneratormodulen typischerweise erfolgt, zu erreichen. Diese Durchströmung mit Arbeitsgas stellt sicher, dass eine gewünschte Zusammensetzung des Arbeitsgases trotz der in dem Plasma erfolgenden Reaktionen aufrechterhalten wird. Zudem wird verhindert, dass Reaktionsprodukte aus dem Plasma unkontrolliert in die Umgebung des Plasmageneratormoduls gelangen. Weiterhin bewirkt die Umströmung der Elektroden der Plasmageneratormodule in den oder die Absaugkanäle hinein eine fortlaufende Kühlung der Elektroden und ihrer dielektrischen Abschirmungen.

An den Absaugkanal kann ein Absauggebläse angeschlossen werden. Von den Gasleitelementen geführtes Arbeitsgas, das zwischen die Elektroden gelangt und durch den Elektrodenabstand hindurch tritt, tritt von dort um die mindestens eine der beiden Elektroden herum in eine schlitzförmige Mündung des Absaugkanals ein, durch den hindurch es von dem Absauggebläse angesaugt wird

Das Arbeitsgas der Plasmageneratormodule kann Luft sein. Die Luft kann an einer den Elektroden abgewandten Seite des Plasmageneratormoduls aus der Umgebung über den Gaszuführkanal zwischen die Gasleitelemente eintreten. Dieses Eintreten kann dabei durch ein aktives Ansaugen der Luft über den an mindestens eine der Elektroden seitlich angrenzenden Absaugkanal mit dem Absauggebläse bewirkt werden.

Das Absauggebläse kann zentral für mehrere Plasmageneratormodule vorgesehen sein. Aber auch jedes einzelne Plasmageneratormodul kann ein solches Absauggebläse aufweisen. Das Absauggebläse kann dabei als auswechselbare Einheit vorgesehen sein, die beispielsweise über eine Steckverbindung an den Luftabsaugkanal angeschlossen ist. Darüber hinaus kann zentral oder an jedem Plasmageneratormodul eine Sensoreinheit zum Erfassen bestimmter Inhaltsstoffe in dem abgesaugten Gas, d. h. der abgesaugten Luft, vorhanden sein. Die Sensoreinheit kann dabei an eine zentrale Auswerteeinrichtung angeschlossen sein, die beispielsweise steuern kann, ob das abgesaugte Gas freigesetzt werden darf oder zunächst noch einer Reinigung bedarf.

In einer Ausführungsform der Plasmageneratormodule mit verstellbarem Elektrodenabstand ist mindestens eine der Elektroden so an einem ersten Ende eines der beiden Gasleitelemente gelagert, dass die mindestens eine der beiden Elektroden zusammen mit dem ersten Ende des anderen der beiden Gasleitelemente gegenüber der anderen der beiden Elektroden und dem anderen der beiden Gasleitelemente in Richtung des Elektrodenabstands verlagerbar ist, wodurch der Elektrodenabstand für die Ausbildung geometrisch unterschiedlicher Entladungsformen auf unterschiedliche Werte einstellbar ist.

In der Ausführungsform der Plasmageneratormodule mit verstellbarem Elektrodenabstand ist also eine solche Einstellbarkeit des seitlichen Elektrodenabstands gegeben, dass unter Verwendung derselben Elektroden geeignete Voraussetzungen für die Ausbildung geometrisch unterschiedlicher Entladungsformen geschaffen werden können. Dabei werden zugleich die Gasleitelemente zum Leiten des Arbeitsgases zwischen die Elektroden modifiziert. Beides wird gleichzeitig dadurch erreicht, dass zumindest eine der beiden Elektroden an dem unteren Ende eines der beiden Gasleitelemente der Gasleiteinrichtung gelagert ist und zusammen mit diesem gegenüber der anderen der Elektroden verlagert wird.

Es versteht sich, dass bei den Plasmageneratormodulen auch beide Elektroden jeweils mit einer dielektrischen Abschirmung versehen sein können, worunter insbesondere zu verstehen ist, dass sie direkt von einem Dielektrikum umschlossen sind. Ebenso sind vorzugsweise auch beide Elektroden an jeweils einem ersten Ende eines der beiden Gasleitelemente so gelagert, dass die beiden Elektroden jeweils zusammen mit dem ersten Ende des jeweiligen Gasleitelements relativ zueinander in Richtung des Elektrodenabstand verlagerbar sind.

In der Ausführungsform der Plasmageneratormodule mit verstellbarem Elektrodenabstand kann die Verlagerbarkeit der jeweiligen Elektrode dadurch realisiert sein, dass das Gasleitelement mit seinem dem ersten Ende gegenüberliegenden zweiten Ende in einem einachsigen Schwenklager an einer Grundstruktur des jeweiligen Plasmageneratormoduls gelagert ist oder starr an der Grundstruktur gelagert ist und selbst ein einachsiges Festkörpergelenk ausbildet, so dass die Elektrode mit dem ersten Ende des Gasleitelements um das Schwenklager oder das Festkörpergelenk herum verschwenkbar ist.

Die oberen Ende der Gasleitelemente liegen in jedem Fall ortsfest an der Grundstruktur des jeweiligen Plasmageneratormoduls, und ein gegenüber der Grundstruktur ortsfester Gaszuführungskanal, der das Arbeitsgas zuführt, kann dort zwischen die beiden Gasleitelemente einmünden.

Um die Elektroden in der Ausführungsform der Plasmageneratormodule mit verstellbarem Elektrodenabstand relativ zueinander zu verlagern, können an Seitenkanten der Gasleitelemente, an denen jeweils eine verlagerbare Elektrode gelagert ist, zwei Schieber angreifen, die bei ihrem Verschieben längs einer quer zu den Elektroden verlaufenden Führung den Elektrodenabstand verändern. Wenn beide Elektroden gegenüber der Grundstruktur verlagerbar sind, können diese Schieber an beiden Gasleitelementen angreifen, und die Schieber können in einer zwischen den Gasleitelementen verlaufenden Hauptrichtung verschoben werden.

Wenn das jeweilige Plasmageneratormodul eine zusätzliche dielektrische Blende aufweist, die aus einer inaktiven Position zwischen den Gasleitelementen in eine aktive Position zwischen den Elektroden und zurück überführbar ist, kann diese dielektrische Blende in der Ausführungsform der Plasmageneratormodule mit verstellbarem Elektrodenabstand an die Schieber gekoppelt sein. Dies bedeutet, dass beim Verschieben der Schieber auf die Elektroden zu, die dielektrische Blende zwischen die Elektroden verschoben wird.

In einer Ausführungsform der Plasmageneratormodule ist die Hochspannungsquelle Teil des jeweiligen Plasmageneratormoduls. Insbesondere in der Ausführungsform des erfindungsgemäßen Plasmageneratormoduls mit verstellbarem Elektrodenabstand kann diese Hochspannungsquelle für die Ausbildung der geometrisch unterschiedlichen Entladungsformen zwischen verschiedenen Betriebsmodi umschaltbar sein, in denen sie verschiedene Wechselhochspannungen an die Elektroden anlegt. Das Anschließen der Hochspannungsquelle der Plasmageneratormodule an eine externe Spannungsquelle kann dann, wenn diese externe Spannungsquelle eine Niederspannungsquelle ist, vorteilhafter Weise ausschließlich über Niederspannungsanschlüsse und -leitungen erfolgen. Das heißt, die Teile, an denen Hochspannung anliegt, sind auf Bereiche des Plasmageneratormoduls beschränkt, die relativ leicht abgrenzbar und isolierbar sind, was bei einer externen Hochspannungsversorgung wesentlich komplexer wäre.

Die Hochspannungsquelle kann insbesondere als auswechselbare Einheit an dem Plasmageneratormodul vorgesehen sein, um sie beispielsweise gegen eine Einheit mit höherer Maximalspannung oder höherer maximaler elektrischer Leistung austauschen zu können.

Die Plasmageneratormodule sind jeweils seitlich längs der und quer zu den Elektroden durch ein Modulgehäuse begrenzt. Dieses Modulgehäuse kann zudem, insbesondere an seinen Seitenwänden, mit Befestigungseinrichtungen versehen sein. Das Modulgehäuse grenzt das Plasmageneratormodul definiert seitlich ab, und es bietet zugleich die Möglichkeit, mehrere Plasmageneratormodule längs der und/oder quer zu den Elektroden zu einem Modularray zusammenzukoppeln. Konkret sind mehrere Plasmageneratormodule mit den Befestigungseinrichtungen aneinander oder an einem gemeinsamen Modulrahmen befestigt. Hierdurch lässt sich die Größe des Bereichs eines Objekts, der in einem bestimmten Zeitraum mit den Plasmamodulen einer Plasmabehandlung unterzogen werden kann, leicht vervielfachen. Dabei besteht keine Gefahr, dass die Behandlung in den unterschiedlichen Teilen dieses Bereichs stark unterschiedlich ausfällt, weil jedes Plasmageneratormodul unabhängig von den ihnen benachbarten Plasmageneratormodulen arbeitet und ein Plasma bereitstellt. Die verschiedenen Plasmageneratormodule des Modularrays können daher auch unterschiedliche Plasmas mit unterschiedlichen Entladungsformen erzeugen, in der Ausführungsform der Plasmageneratormodule mit verstellbarem Elektrodenabstand selbst dann, wenn alle Plasmageneratormodule grundsätzlich baugleich sind.

Bei einer erfindungsgemäßen Verwendung eines erfindungsgemäßen Modularrays werden für die Ausbildung geometrisch unterschiedlicher Entladungsformen räumlich nebeneinander unterschiedliche Werte des Elektrodenabstand eingestellt. In der Ausführungsform der Plasmageneratormodule mit verstellbarem Elektrodenabstand können unterschiedliche Werte des Elektrodenabstand auch zeitlich nacheinander an einem selben Plasmageneratormodul eingestellt werden. Die unterschiedlichen Entladungsformen umfassen dabei insbesondere eine direkte dielektrisch behinderte Entladung zwischen den beiden Elektroden einerseits und einem ihnen gegenüberliegenden Objekt andererseits, eine dielektrisch behinderte Entladung zwischen den beiden Elektroden, die als Plasmajet auf ein ihnen gegenüberliegendes Objekt ausgeblasen wird, und eine koplanare Gleitentladung zwischen einer der beiden Elektroden und einem den Elektroden gegenüberliegenden Objekt sowie zwischen dem Objekt und der anderen der beiden Elektroden. Die Hochspannungsquelle wird für die unterschiedlichen Entladungsformen in unterschiedlicher Weise betrieben, wobei bei der direkten dielektrisch behinderten Entladung eine gleiche Wechselspannung an den beiden Elektroden gegenüber dem Objekt oder Erde angelegt wird und wobei bei dem Plasmajet und der koplanaren Gleitentladung eine Wechselspannung zwischen den beiden Elektroden angelegt wird. In diesem Zusammenhang ist der Begriff Wechselspannung sehr breit zu verstehen. Insbesondere soll er auch zeitliche Verläufe der anliegenden Spannung mit kurzzeitigen Spannungspulsen und nicht symmetrischem Spannungsverlauf umfassen. Welcher zeitlicher Verlauf der Wechselspannung für die verschiedenen Entladungsformen besonders günstig ist und welche genauen Abstände zwischen den Elektroden für die verschiedenen Entladungsformen sinnvollerweise eingestellt werden, ist dem Fachmann grundsätzlich bekannt. Der Fachmann kann diese Werte aber auch durch einfaches Ausprobieren des jeweiligen Plasmageneratormoduls ermitteln.

Vorteilhafte Weiterbildungen der Erfindung ergeben sich aus den Patentansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen. Ohne dass hierdurch der Gegenstand der beigefügten Patentansprüche verändert wird, gilt hinsichtlich des Offenbarungsgehalts der ursprünglichen Anmeldungsunterlagen und des Patents Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von zwei Gasleitelementen die Rede ist, ist dies so zu verstehen, dass genau zwei Gasleitelemente, drei Gasleitelemente oder mehr Gasleitelemente vorhanden sind. Diese Merkmale können durch andere Merkmale ergänzt werden oder die einzigen Merkmale sein, aus denen das jeweilige Erzeugnis besteht.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: ist eine perspektivische Darstellung eines Plasmageneratormoduls.
- **Fig. 2**: ist eine perspektivische Darstellung einer Baugruppe des Plasmageneratormoduls gemäß Fig. 1.
- **Fig. 3**: ist eine Seitenansicht der Baugruppe gemäß Fig. 2 ergänzt um einen Schieber zur Einstellung eines seitlichen Elektrodenabstands.
- **Fig. 4**: illustriert schematisch drei Entladungsformen bei einer mithilfe des Plasmageneratormoduls gemäß den Fig. 1 bis 3 hervorgerufenen dielektrisch behinderten Entladung.
- **Fig. 5**: ist ein Schnitt durch eine weitere Ausführungsform eines Plasmageneratormoduls.
- **Fig. 6**: illustriert den Aufbau eines Modularrays aus mehreren Plasmageneratormodulen gemäß Fig. 1.
- **Fig. 7**: illustriert den Aufbau eines größeren Modularrays aus mehreren Plasmageneratormodulen gemäß Fig. 1.
- **Fig. 8**: ist eine Fig. 3 entsprechende Seitenansicht der Baugruppe einer anderen Ausführungsform des Plasmageneratormoduls gemäß Fig. 1; und
- **Fig. 9**: illustriert den Aufbau eines Modularrays aus mehreren Plasmageneratormodulen mit anderen Befestigungseinrichtungen als bei den Modularrays gemäß Fig. 6 und 7.

### FIGURENBESCHREIBUNG

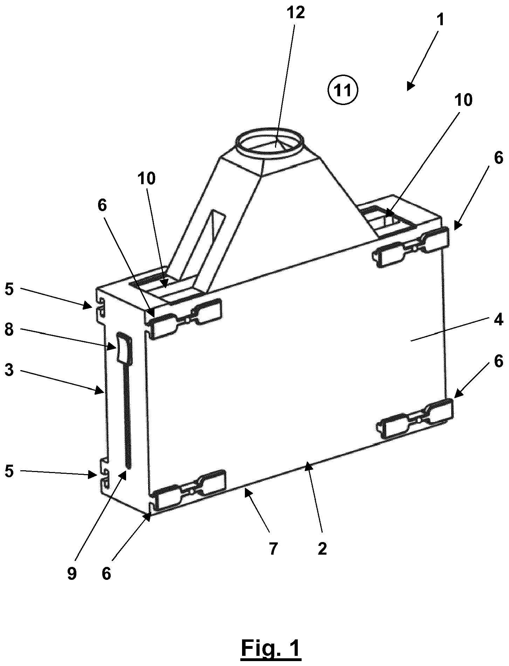

Das in **Fig. 1** in einer perspektivischen Ansicht dargestellte Plasmageneratormodul 1 weist ein Modulgehäuse 2 auf. Seitenwände 3 und 4 des Modulgehäuses 2 sind mit komplementären Befestigungseinrichtungen 5 und 6 versehen, die für ein Koppeln mehrerer gleicher Plasmageneratormodule 1 aneinander ausgebildet sind. An einer Unterseite 7 des Gehäuses 2 sind zwei hier nicht sichtbare Elektroden angeordnet, deren seitlicher Elektrodenabstand mithilfe von zwei Schiebern 8 einstellbar ist, von denen hier nur einer sichtbar ist und die in schlitzförmigen Führungen 9 in dem Gehäuse 2 vertikal geführt sind. An der Oberseite des Gehäuses 2 tritt an zwei Stellen ein Gaszuführkanal 10 in das Modulgehäuse 2 ein, über den Luft aus der Umgebung 11 in das Gehäuse 2 eingesaugt wird. Das Einsaugen erfolgt über einen Absaugkanal 12, wenn an diesen ein hier nicht dargestelltes Absauggebläse angeschlossen wird.

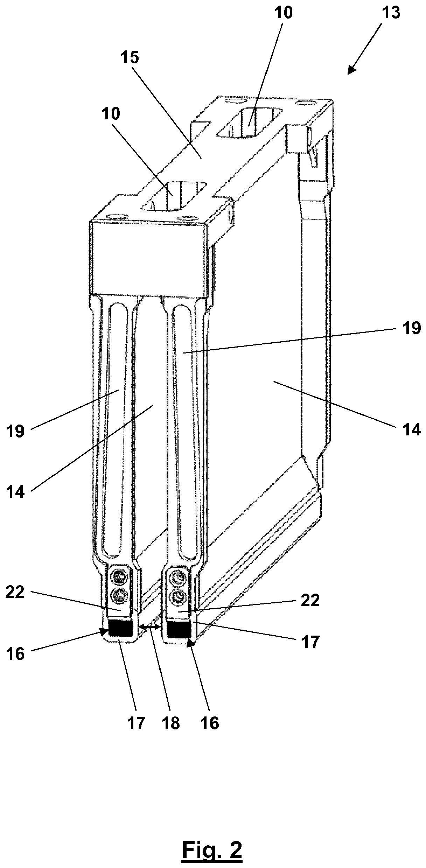

**Fig. 2** zeigt eine Baugruppe 13 des Plasmageneratormoduls 1 gemäß Fig. 1, die im inneren des Gehäuses 2 gemäß Fig. 1 angeordnet ist. Oben in eine ortsfeste Grundstruktur 15 der Baugruppe 13 tritt der Gaszufuhrkanal 10 in zwei Bereichen ein, und diese Bereiche münden zwischen zwei Gasleitelemente 14. Die Gasleitelemente 14 sind mit oberen Enden ortsfest an der Grundstruktur 15 gelagert, und an ihren unteren Enden ist jeweils eine der beiden Elektroden 16 gelagert. Die Elektroden 16 werden dazu an den unteren Enden der Gasleitelemente 14 durch Spannpratzen 22 gehalten. Jede der beiden Elektroden 16 ist mit einer dielektrischen Abschirmung 17 versehen. Gegenüber der Grundstruktur 15 sind die linear langgestreckten und parallel zueinander ausgerichteten Elektroden 16 nicht ortsfest. Vielmehr sind die Gasleitelemente 14 im Sinne von einachsigen Festkörpergelenken verformbar. Dies erlaubtes, einen seitlichen Elektrodenabstand 18 zu variieren, indem die Elektroden 16 um die Festkörpergelenke herum verschwenkt werden. Unabhängig von dem eingestellten Elektrodenabstand 18 führen die Gasleitelemente 14 das über den Gaszuführkanal 10 zwischen die Gasleitelemente 14 eintretende Arbeitsgas den Elektroden 16 zu.

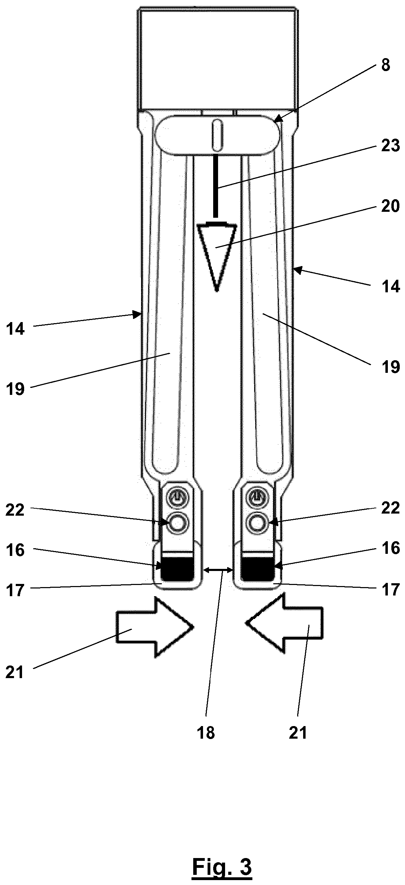

Zur kontrollierten Variation des seitlichen Elektrodenabstands sind die Schieber 8 vorgesehen, die jeweils mit Betätigungselementen in Führungsbahnen 19 eingreift, die an den Seitenkanten der Gasleitelemente 14 vorgesehen sind. **Fig. 3** illustriert, wie ein Herunterschieben der Schieber 8 in Richtung eines Pfeils 20, die nach unten zunächst V-förmig auseinanderlaufenden Führungsbahnen 19 und damit auch die Elektroden 16 in Richtung von zwei Pfeilen 21 zusammenführt. Dadurch wird der Elektrodenabstand 18 verringert. Die Führungsbahnen 19 können aber auch einen anderen als geradlinigen Verlauf aufweisen, so dass sich der Elektrodenabstand beim Herunterschieben der Schieder 8 nicht monoton ändert. Mit den Schiebern 8 wird zudem eine dielektrische Blende 23 nach unten bis in den seitlichen Elektrodenabstand 18 zwischen den Elektroden 16 bewegt.

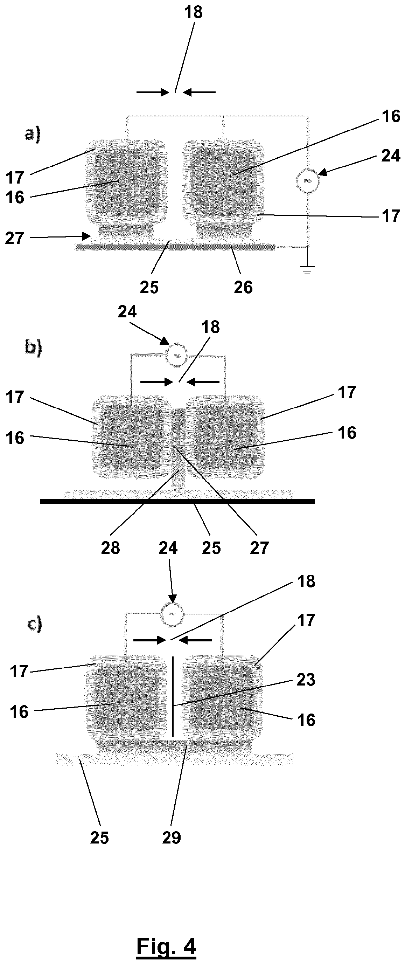

**Fig. 4** illustriert verschiedene Entladungsformen, wie sie beim Generieren eines physikalischen Plasmas bei Atmosphärendruck mit dem Plasmageneratormodul gemäß den Fig. 1 bis 3 hervorgerufen werden können. Gemäß **Fig. 4a****)** wird bei einem beliebigen Elektrodenabstand 18 derselbe Pol einer Wechselhochspannung einer Hochspannungsquelle 24 an beide Elektroden 16 angelegt. Die Wechselhochspannung wird dabei von der Hochspannungsquelle 24 zum Beispiel gegenüber Erde erzeugt und ein zu behandelndes Objekt 25 wird auf eine geerdete Auflage 26 aufgelegt. Unter diesen Voraussetzungen bildet sich eine direkte dielektrisch behinderte Entladung 27 zwischen den Elektroden 26 und dem Objekt 25 aus.

Gemäß **Fig. 4b****)** wird bei relativ kleinem Elektrodenabstand 18 mit der Hochspannungsquelle 24 eine Wechselhochspannung zwischen den Elektroden 16 angelegt, die zu einer direkten dielektrisch behinderten Entladung 27 zwischen den Elektroden 16 führt. Das durch die direkte dielektrisch behinderte Entladung 27 erzeugte physikalische Plasma wird dabei in Form eines Plasmajets 28 zwischen den Elektroden 16 hindurch auf das zu behandelnde Objekt 25 ausgeblasen.

Gemäß **Fig. 4c****)** wird bei relativ großem Elektrodenabstand 18 und/oder zwischen den Elektroden 16 angeordneter dielektrischer Blende 23 mit der Hochspannungsquelle 24 eine Wechselhochspannung zwischen den Elektroden 16 angelegt. Der Elektrodenabstand 18 bzw. die dielektrische Blende verhindert dabei eine direkte dielektrisch behinderte Entladung zwischen den Elektroden 16. Vielmehr kommt es zur Ausbildung einer koplanaren Gleitentladung 29 jeweils zwischen einer der Elektroden 16 und dem zu behandelnden Objekt 25. Bei dem Plasmageneratormodul 1 gemäß Fig. 1 bis 3 ist der für die Entladungsformen 4a) bis c) jeweils optimale Elektrodenabstand 18 einstellbar. Darüber hinaus kann auch die Hochspannungsquelle 24 in das Plasmageneratormodul integriert oder an dieses angesetzt sein, um jeweils in den Betriebsmodus geschaltet zu werden, der für die jeweilige Entladungsformen bezüglich der ausgegebenen Wechselhochspannung passend ist. Dabei kann das Schalten der Hochspannungsquelle in den jeweiligen Betriebsmodus durch das Verschieben des Schiebers 8 erfolgen.

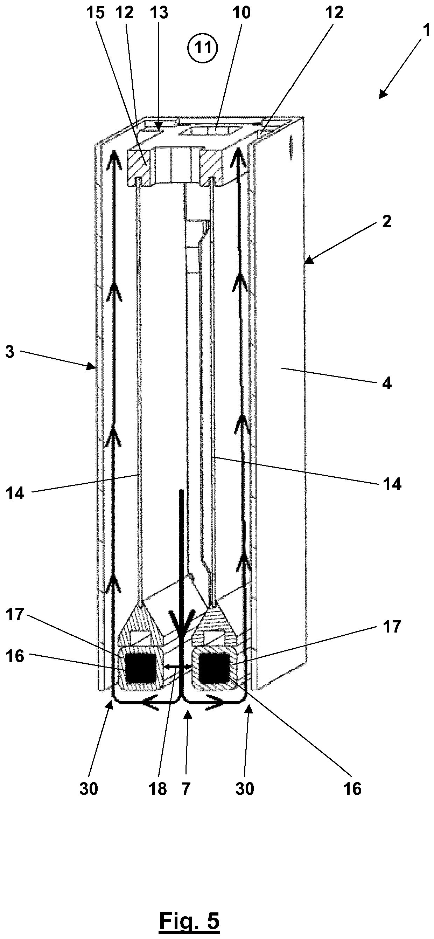

**Fig. 5** erläutert anhand eines Schnitts durch eine andere Ausführungsform des Plasmageneratormoduls 1 die Führung des Arbeitsgases Luft durch das Modulgehäuse 2 und dabei zwischenzeitlich um die Elektroden 16 an der Unterseite 7 des Gehäuses 2 herum. Das Arbeitsgas Luft tritt aus der Umgebung 11 durch den Gaszuführkanal 10 zwischen die Gasleitelemente 14 ein und gelangt von diesen geführt von oben zwischen die Elektroden 16. Es tritt durch den Elektrodenabstand 18 hindurch und läuft von dort um die Elektroden 16 herum in schlitzförmige Mündungen 30 des Absaugkanals 12 ein, durch den hindurch es von dem nicht dargestellten Absauggebläse angesaugt wird.

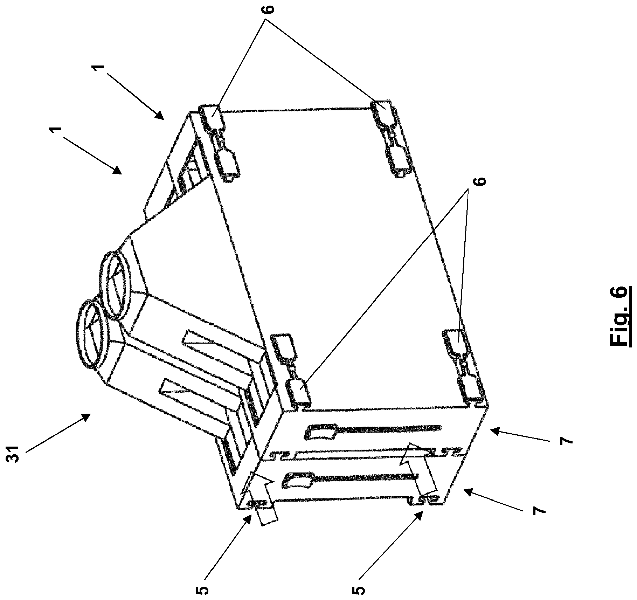

**Fig. 6** skizziert, wie mehrere Plasmageneratormodule 6 mithilfe ihrer Befestigungseinrichtungen 5 und 6 hier in Querrichtung zu ihren an ihren Unterseiten 7 angeordneten Elektroden aneinander gekoppelt werden können, um ein bezüglich der mit ihm durchführbaren Plasmabehandlung eines Objekts größerflächigeres Modularray 31 auszubilden.

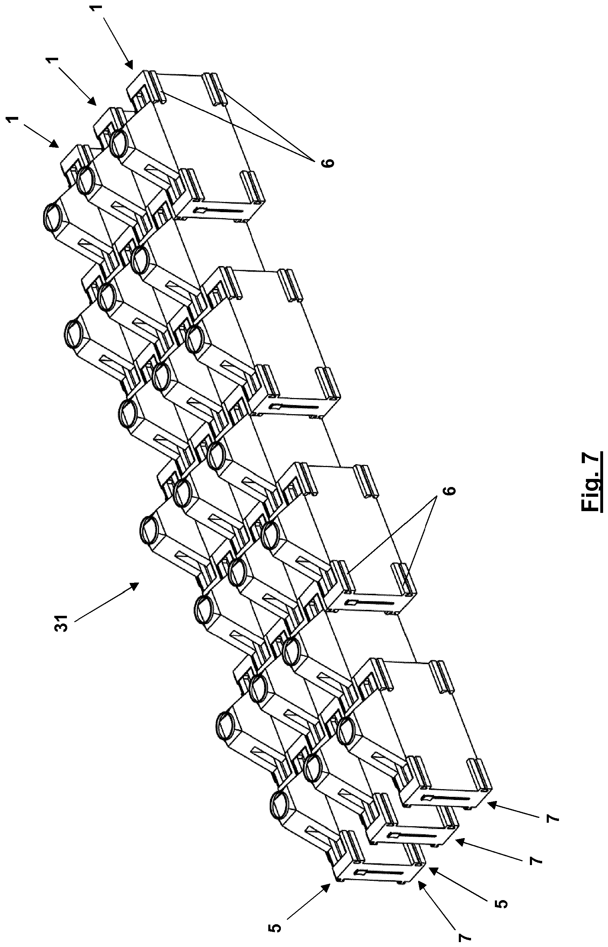

**Fig. 7** skizziert, wie mit Hilfe der Befestigungseinrichtungen 5 und 6 mehrere Plasmageneratormodule 1 auch versetzt zueinander zu einem Modularray 31 aneinander gekoppelt werden können. Während Fig. 7 ein flächiges Generatormodul 31 zeigt, bei dem in Querrichtung jeweils drei Generatormodule hintereinander angeordnet sind, die sich beidseitig mit drei benachbarten Generatormodulen teilweise überlappen, kann auch nur eine hin- und her springende oder diagonale Reihe von sich teilweise überlappenden Generatormodulen vorgesehen sein. Die teilweise Überlappung der Generatormodule stellt sicher, dass das physikalische Plasma an der Unterseite des Modularrays lückenlos über die gesamte Breite des Modularrays generiert wird.

Während die Fig. 6 und 7 die Ausbildung eines Modularrays 31 aus gleichen Plasmageneratormodulen 6 in der Ausführungsform mit verstellbarem Elektrodenabstand zeigen, kann ein solches Modularray auch verschieden ausgebildete Plasmageneratormodule 6 aufweisen, um innerhalb des Modularrays 31 geometrisch unterschiedliche Entladungsformen der dielektrisch behinderten Entladung 27 bei Atmosphärendruck auszubilden.

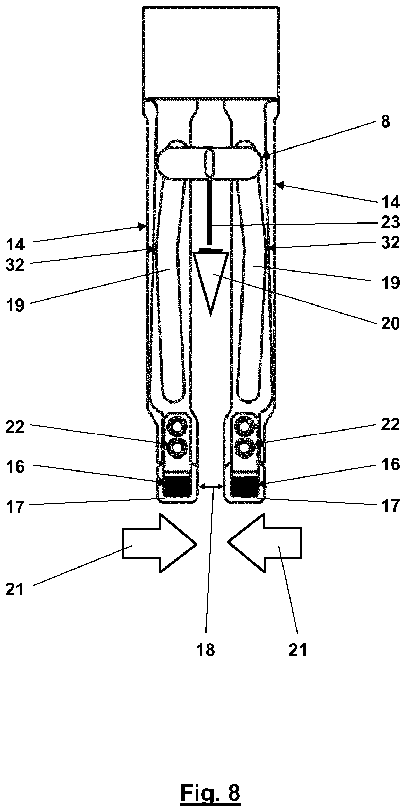

Die in **Fig. 8** gezeigte alternative Ausführungsform der Baugruppe 13 weist einen anderen Verlauf der Führungsbahn 19 auf, so dass der minimale Elektrodenabstand 18 dann erreicht wird, wenn sich der Schieber 8 auf Höhe von nach außen gerichteten Knicken 32 der Führungsbahnen 19 befindet. Dadurch vergrößert sich der Elektrodenabstand 18 wieder, wenn mit dem Schieber 8 die dielektrische Blende 23 zwischen die Elektroden 16 verfahren wird, um beste Voraussetzungen für eine koplanare Gleitentladung 29 gemäß Fig. 4c) zu schaffen.

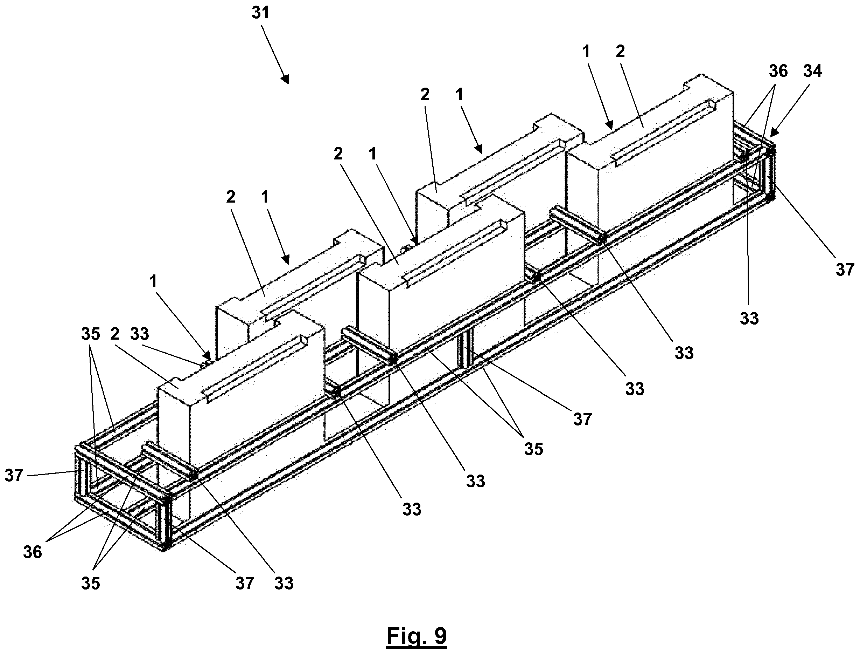

**Fig. 9** zeigt ein Modularray 31, das auf Plasmageneratormodulen 1 mit anders gestaltetem Modulgehäuse 2 basiert, als die Modularrays gemäß den Figuren 6 und 7. Gemäß Fig. 9 sind Befestigungselemente 33 an den Modulgehäusen 2 in Form von sich auf etwa halber Höhe über die Stirnseiten der Modulgehäuse 2 hinweg erstreckenden Querträgern vorgesehen. Mithilfe dieser Befestigungseinrichtungen 33 sind die Plasmageneratormodule 1 in einen Modulrahmen 34 eingehängt, der aus Längsstreben 35, Querstreben 36 und Vertikalstreben 37 aufgebaut ist. Dabei werden die Modulgehäuse 2 der Plasmageneratormodule 1 durch die Längsstreben 35 hier in zwei parallelen Vertikalebenen geführt, in denen sie versetzt zueinander angeordnet sind. Auf den oberen Längsstreben 35 liegen die Plasmageneratormodule 1 mit ihren Befestigungseinrichtungen 33 auf. Längs der Längsstreben 35 sind die Plasmageneratormodule 1 frei positionierbar, bis sie mit ihren Befestigungseinrichtungen 33 aneinanderstoßen.

### BEZUGSZEICHENLISTE

- 1: Plasmageneratormodul
- 2: Modulgehäuse
- 3: Seitenwand
- 4: Seitenwand
- 5: Befestigungseinrichtung
- 6: Befestigungseinrichtung
- 7: Unterseite
- 8: Schieber
- 9: Führung
- 10: Gaszuführkanal
- 11: Umgebung
- 12: Absaugkanal
- 13: Baugruppe
- 14: Gasleitelement
- 15: Grundstruktur
- 16: Elektrode
- 17: dielektrische Abschirmung
- 18: Elektrodenabstand
- 19: Führungsbahn
- 20: Pfeil
- 21: Pfeil
- 22: Spannpratze
- 23: dielektrische Blende
- 24: Hochspannungsquelle
- 25: Objekt
- 26: Auflage
- 27: dielektrisch behinderte Entladung
- 28: Plasmajet
- 29: koplanare Gleitentladung
- 30: schlitzförmige Mündung
- 31: Modularray
- 32: Knick
- 33: Befestigungseinrichtung
- 34: Modulrahmen
- 35: Längsstrebe
- 36: Querstrebe
- 37: Vertikalstrebe

## Patentansprüche

1. Modularray (31) aus mehreren Plasmageneratormodulen (1) zum Generieren eines physikalischen Plasmas durch dielektrisch behinderte Entladung (27) bei Atmosphärendruck,
wobei die Plasmageneratormodule (1) jeweils
- zwei linear langgestreckte Elektroden (16),
- von denen mindestens eine mit einer dielektrischen Abschirmung versehen ist und
- die in einem seitlichen Elektrodenabstand (18) parallel zueinander verlaufen;
- Anschlusseinrichtungen zum Anschließen der Elektroden (16) an eine Hochspannungsquelle (24) und
- zwei flächigen Gasleitelementen zum Leiten von Arbeitsgas zwischen die Elektroden (16) aufweisen,
wobei an mindestens eine der beiden Elektroden (16) des jeweiligen Plasmageneratormoduls (1), auf ihrer der anderen Elektrode (16) abgekehrten Außenseite eine schlitzförmige Mündung (30) eines Absaugkanal (12) angrenzt, wobei sich der Absaugkanal (12) auf einer Außenseite des an die mindestens eine der beiden Elektroden (16) anschließenden Gasleitelements über dieses Gasleitelement erstreckt,
wobei die Plasmageneratormodule (1) jeweils seitlich längs der und quer zu den Elektroden (16) durch ein Modulgehäuse (2) begrenzt sind,
wobei die Elektroden (16) jeweils an einer Unterseite (7) des Modulgehäuses (2) angeordnet sind,
wobei die Modulgehäuse (2) der mehreren Plasmageneratormodule (1) mit Befestigungseinrichtungen (5, 6, 33) versehen sind und mittels der Befestigungseinrichtungen (5, 6, 33) aneinander oder an einem gemeinsamen Modulrahmen (34) befestigt sind, und
wobei die mehreren Plasmageneratormodule (6) längs der und quer zu den Elektroden (16) nebeneinander und derart versetzt zueinander angeordnet sind, dass sie sich längs der Elektroden (16) teilweise überlappen.

2. Modularray (31) nach Anspruch 1, **dadurch gekennzeichnet, dass** verschiedene der Plasmageneratormodule (1) für die Ausbildung geometrisch unterschiedlicher Entladungsformen der dielektrisch behinderten Entladung (27) bei Atmosphärendruck ausgebildet sind, indem unterschiedliche Werte des Elektrodenabstands (18) eingestellt sind.

3. Modularray (31) nach Anspruch 1 oder 2, **dadurch gekennzeichnet, dass** die Befestigungseinrichtungen (5, 6) an Seitenwänden des Modulgehäuses (2) ausgebildet sind.

4. Modularray (31) nach einem der vorhergehenden Ansprüche, **dadurch gekennzeichnet, dass** mindestens eine der beiden Elektroden (16) des jeweiligen Plasmageneratormoduls (1) so an einem ersten Ende eines der beiden Gasleitelemente gelagert ist, dass die mindestens eine der beiden Elektroden (16) zusammen mit dem ersten Ende des einen der beiden Gasleitelemente gegenüber der anderen der beiden Elektroden (16) und dem anderen der beiden Gasleitelemente in Richtung des Elektrodenabstands (18) verlagerbar ist, wodurch der Elektrodenabstand (18) für die Ausbildung geometrisch unterschiedlicher Entladungsformen der dielektrisch behinderten Entladung (27) bei Atmosphärendruck auf unterschiedliche Werte einstellbar ist.

5. Modularray (31) nach Anspruch 4, **dadurch gekennzeichnet, dass** das eine der beiden Gasleitelemente mit seinem dem ersten Ende gegenüberliegenden zweiten Ende in einem einachsigen Schwenklager an einer Grundstruktur (15) des jeweiligen Plasmageneratormoduls (1) gelagert ist oder starr an der Grundstruktur (15) gelagert ist und selbst ein einachsiges Festkörpergelenk ausbildet, wobei die eine der beiden Elektroden (16) mit dem ersten Ende des einen der beiden Gasleitelemente um das Schwenklager oder das Festkörpergelenk herum verschwenkbar ist, wobei, optional, ein gegenüber der Grundstruktur (15) des jeweiligen Plasmageneratormoduls (1) ortsfester Gaszufuhrkanal zwischen die beiden Gasleitelemente einmündet.

6. Modularray (31) nach einem der Ansprüche 4 und 5, **dadurch gekennzeichnet, dass** beide Elektroden (16) des jeweiligen Plasmageneratormoduls (1) an jeweils einem ersten Ende eines der beiden Gasleitelemente so gelagert sind, dass die beiden Elektroden (16) jeweils zusammen mit dem ersten Ende des jeweiligen Gasleitelements relativ zueinander in Richtung des Elektrodenabstands (18) verlagerbar sind.

7. Modularray (31) nach einem der Ansprüche 4 bis 6, **dadurch gekennzeichnet, dass** an Seitenkanten der Gasleitelemente, an denen jeweils eine verlagerbare Elektrode (16) des jeweiligen Plasmageneratormoduls (1) gelagert ist, zwei Schieber (8) des jeweiligen Plasmageneratormoduls (1) angreifen, die beim Verschieben längs einer quer zu den Elektroden (16) verlaufenden Führung (9) den Elektrodenabstand (18) des jeweiligen Plasmageneratormoduls (1) verändern.

8. Modularray (31) nach einem der vorhergehenden Ansprüche, **dadurch gekennzeichnet, dass** eine dielektrische Blende (23) des jeweiligen Plasmageneratormoduls (1) aus einer inaktiven Position zwischen den Gasleitelementen in eine aktive Position zwischen den Elektroden (16) des jeweiligen Plasmageneratormoduls (1) und zurück überführbar ist.

9. Modularray (31) nach Anspruch 8 soweit rückbezogen auf Anspruch 7, **dadurch gekennzeichnet, dass** die dielektrischen Blende des jeweiligen Plasmageneratormoduls (1) an die Schieber (8) des jeweiligen Plasmageneratormoduls (1) gekoppelt ist.

10. Modularray (31) nach einem der vorhergehenden Ansprüche, **dadurch gekennzeichnet, dass** die Hochspannungsquelle (24) Teil des jeweiligen Plasmageneratormoduls (1) ist.

11. Modularray (31) nach Anspruch 10 soweit direkt oder indirekt rückbezogen auf Anspruch 4, **dadurch gekennzeichnet, dass** die Hochspannungsquelle (24) des jeweiligen Plasmageneratormoduls (1) für die Ausbildung der geometrisch unterschiedlichen Entladungsformen zwischen verschiedenen Betriebsmodi umschaltbar ist, in denen sie verschiedenen Wechselhochspannungen an die Elektroden (16) des jeweiligen Plasmageneratormoduls (1) anlegt, und wobei die Hochspannungsquelle (24) ausschließlich über Niederspannungsanschlüsse und - leitungen an eine externe Niederspannungsquelle anschließbar ist.

12. Verwendung eines Modularrays (31) nach einem der vorhergehenden Ansprüche, **dadurch gekennzeichnet, dass** an den Absaugkanal (12) des jeweiligen Plasmageneratormoduls (1) ein Absauggebläse angeschlossen ist und dass von den Gasleitelementen (14) geführtes Arbeitsgas zwischen die Elektroden (16) des jeweiligen Plasmageneratormoduls (1) gelangt, durch den Elektrodenabstand (18) hindurch tritt und von dort um die mindestens eine der beiden Elektroden (16) herum in eine schlitzförmige Mündung (30) des Absaugkanals (12) eintritt, durch den hindurch es von dem Absauggebläse angesaugt wird.

13. Verwendung nach Anspruch 12, **dadurch gekennzeichnet, dass** Luft als Arbeitsgas aus einer Umgebung (11) des jeweiligen Plasmageneratormoduls (1) durch einen Gaszuführkanal (10) zwischen die Gasleitelemente (14) eintritt.

14. Verwendung eines Modularrays (31) nach einem der Ansprüche 1 bis 11 oder Verwendung nach Anspruch 12 oder 13, **dadurch gekennzeichnet, dass** zeitlich nacheinander oder räumlich nebeneinander geometrisch unterschiedlicher Entladungsformen der dielektrisch behinderten Entladung (27) bei Atmosphärendruck ausgebildet werden, wobei die unterschiedlichen Entladungsformen
- eine direkte dielektrisch behinderte Entladung (27) zwischen den beiden Elektroden (16) einerseits und einem ihnen gegenüberliegenden Objekt (25) andererseits,
- eine dielektrisch behinderte Entladung (27) zwischen den beiden Elektroden (16), die als Plasmajet (28) auf ein ihnen gegenüberliegendes Objekt (25) ausgeblasen wird, und
- eine koplanare Gleitentladung (29) zwischen einer der beiden Elektroden (16) und einem den Elektroden (16) gegenüberliegenden Objekt (25) sowie zwischen dem Objekt (25) und der anderen der beiden Elektroden (16)
umfassen,
wobei für die Ausbildung der geometrisch unterschiedlichen Entladungsformen unterschiedliche Werte des Elektrodenabstands (18) eingestellt werden.

15. Verwendung nach Anspruch 14, **dadurch gekennzeichnet, dass** die Hochspannungsquellen (24) für die unterschiedlichen Entladungsformen in unterschiedlichen Betriebsmodi betrieben wird/werden, wobei
- bei der direkten dielektrisch behinderten Entladung (27) eine gleiche Wechselspannung an den beiden Elektroden (16) gegenüber dem Objekt (25) oder Erde angelegt wird, und
- bei dem Plasmajet (28) und der koplanaren Gleitentladung (29) eine Wechselspannung zwischen den beiden Elektroden (16) angelegt wird.

## Claims

1. Module array (31) of a plurality of plasma generator modules (1) for generating a physical plasma by dielectric barrier discharge (27) at atmospheric pressure,
wherein the plasma generator modules each comprise
- two linearly elongated electrodes (16),
- at least one of which is provided with a dielectric barrier and
- which extend parallel to one another at a lateral electrode distance (18);
- connection devices for connecting the electrodes (16) to a high voltage source (24); and
- two 2-dimensional gas-guiding elements configured for guiding a working gas between the electrodes (16);
wherein a slit-shaped mouth (30) of a suction channel (12) adjoins at least one of the two electrodes (16) of the respective plasma generator module (1) at its outer side (16) facing away from the other electrode (16), wherein the suction channel (12), at an outer side of the gas-guiding element connecting to the at least one of the two electrodes (16), extends over this gas-guiding element,
wherein the plasma generator modules (1) are each delimited by a module housing (2) laterally along the electrodes and transversely to the electrodes (16),
wherein the electrodes (16) are each arranged at a bottom side (7) of the module housing (2),
wherein the module housings (2) of the plurality of plasma modules (1) are provided with fixation devices (5, 6, 33) and fixed to one another or to a common module frame (34) by means of the fixation devices (5, 6, 33), and
wherein the plurality of plasma modules (6) are arranged side by side along the electrodes (16) and transversely to the electrodes (16), and offset in such a way that they partially overlap along the electrodes (16).

2. Module array (31) of claim 1, **characterized in that** different plasma generator modules (1) are configured for the generation of geometrically different discharge forms of the dielectric barrier discharge (27) at atmospheric pressure **in that** different values of the electrode distance (18) are adjusted.

3. Module array (31) of claim 1 or 2, **characterized in that** the fixation devices (5, 6) are formed at lateral walls of the module housing (2) .

4. Module array (31) of any of the preceding claims, **characterized in that** at least one of the two electrodes (16) of the respective plasma generator module (1) is mounted to a first end of one of the two gas-guiding elements in such a way that the at least one of the two electrodes (16), together with the first end of the one of the two gas-guiding elements, is movable with regard to the other of the two electrodes (16) and the other of the two gas-guiding elements in the direction of the electrode distance (18), such that the electrode distance (18) is adjustable to different values for the generation of geometrically different discharge forms of the dielectric barrier discharge (27) at atmospheric pressure.

5. Module array (31) of claim 4, **characterized in that** the one of the two gas-guiding elements, with its second end opposite to its first end, is mounted to a base structure (15) of the respective plasma generator module (1) in a single swivel axis swivel joint, or is rigidly mounted to the base structure (15) and forms a single swivel axis solid body joint by itself, wherein the one of the two electrodes (16), together with the first end of the one of the two gas-guiding elements can be swiveled about the swivel joint or the solid body joint, wherein, optionally, a gas supply channel, which is spatially fixed with regard to the base structure (15) of the respective plasma module (1) discharges between the two gas-guiding elements.

6. Module array (31) of any of the claims 4 and 5, **characterized in that** both electrodes (16) of the respective plasma generator module (1) are each mounted to a first end of one of the two gas-guiding elements in such a way that the two electrodes (16), each together with the first end of the respective gas-guiding elements, are movable with regard to one another in the direction of the electrode distance (18).

7. Module array (31) of any of the claims 4 to 6, **characterized in that** two sliders (8) of the respective plasma generator module (1) engage lateral edges of the gas-guiding elements to which a movable electrode (16) of the respective plasma generator module (1) is mounted each, the sliders (8), upon being shifted along guides (9) extending transversally to the electrodes (16), altering the electrode distance (18) of the respective plasma generator module (1).

8. Module array (31) of any of the preceding claims, **characterized in that** a dielectric screen (23) of the respective plasma generator module (1) is transferable forth and back between an inactive position between the gas-guiding elements and an active position between the electrodes (16) of the respective plasma generator module (1).

9. Module array (31) of claim 8 so far as dependent on claim 7, **characterized in that** the dielectric screen of the respective plasma generator module (1) is coupled to the sliders (8) of the respective plasma generator module (1).

10. Module array (31) of any of the preceding claims, **characterized in that** the high voltage source (24) is a part of the respective plasma generator module (1).

11. Module array (31) of claim 10 so far as directly or indirectly dependent on claim 4, **characterized in that** the high voltage source (24) of the respective plasma generator module (1), for the generation of the geometrically different discharge forms, is switchable between different operation modes, in which it applies different alternating high voltages to the electrodes (16) of the respective plasma generator module (1), and wherein the high voltage source (24) is exclusively connectable to an external low voltage source via low voltage connectors and lines.

12. Use of a module array (31) of any of the preceding claims, **characterized in that** a suction fan is connected to the suction channel (12) of the respective plasma generator module (1), and that a working gas guided by the gas-guiding elements (14) gets between the electrodes (16) of the respective plasma generator module (1), passes through the electrode distance (18) and gets from there around the at least one of the two electrodes (16) into the slit-shaped mouth (30) of the suction channel (12) through which it is sucked-in by the suction fan.

13. Use of claim 12, **characterized in that** air as the working gas, out of a surrounding (11) of the respective plasma generator module (1), enters through a gas supply channel (10) between the gas-guiding elements (14).

14. Use of a module array (31) of any of the claims 1 to 11 or use of claims 12 or 13, **characterized in that** temporally one after the other, or spatially one next to the other geometrically different discharge forms of the dielectric barrier discharge (27) at atmospheric pressure are generated, wherein the different discharge forms include
- a direct dielectric barrier discharge (27) between the two electrodes (16), on the one hand, and an object (25) facing them, on the other hand,
- a dielectric barrier discharge (27) between the two electrodes (16), which is blown as a plasma jet (28) onto an object facing them, and
- a coplanar sliding discharge (29) between one of the two electrodes (16) and an object (25) facing the electrodes (16) as well as between the object (25) and the other of the two electrodes (16),
wherein different values of the electrode distance (18) are adjusted for the generation of the geometrically different discharge forms.

15. Use of claim 14, **characterized in that** the high voltage sources (24), for the different discharge forms, is/are operated in different operation modes, wherein,
- in the direct dielectric barrier discharge (27), same alternating voltages with respect to the object (25) or earth are applied to the two electrodes (16), and,
- in the plasma jet (28) and the coplanar sliding discharge (29), an alternating voltage is applied between the two electrodes (16).

## Revendications

1. Matrice de modules (31) constituée de plusieurs modules générateurs de plasma (1) pour la génération d'un plasma physique par décharge rendue incomplète par voie diélectrique (27) à la pression atmosphérique, dans laquelle les modules générateurs de plasma (1) comprennent chacun
- deux électrodes linéaires allongées (16),
- dont au moins une est munie d'un écran diélectrique et
- qui s'étendent parallèlement entre elles avec une distance latérale (18) ;
- des dispositifs de raccordement pour le raccordement des électrodes (16) à une source de haute tension (24) et
- deux éléments conducteurs de gaz plats pour le guidage d'un gaz de travail entre les électrodes (16),
dans laquelle, sur au moins une des deux électrodes (16) du module générateur de plasma (1) respectif, sur son côté externe opposé à l'autre électrode (16), se trouve une embouchure en forme de fente (30) d'un canal d'aspiration (12), dans laquelle le canal d'aspiration (12) s'étend, sur un côté externe de l'élément conducteur de gaz se raccordant à au moins une des deux électrodes (16), au-dessus de cet élément conducteur de gaz,
dans laquelle les modules générateurs de plasma (1) sont délimités chacun latéralement, le long et transversalement par rapport aux électrodes (16), par un boîtier de module (2),
dans laquelle les électrodes (16) sont disposées chacune sur un côté inférieur (7) du boîtier de module (2),
dans laquelle les boîtiers de modules (2) des plusieurs modules générateurs de plasma (1) sont munis de dispositifs de fixation (5, 6, 33) et sont fixés, au moyen des dispositifs de fixation (5, 6, 33), les uns aux autres ou à un châssis de module commun (34) et
dans laquelle les plusieurs modules générateurs de plasma (6) sont juxtaposés le long et transversalement par rapport aux électrodes (16) et disposés de manière décalée entre eux de façon à se superposer partiellement le long des électrodes (16).

2. Matrice de modules (31) selon la revendication 1, **caractérisée en ce que** certains des modules générateurs de plasma (1) sont conçus pour la formation de différentes formes géométriques de la décharge rendue incomplète par voie diélectrique (27) à la pression atmosphérique, en ajustant différentes valeurs de la distance entre les électrodes (18).

3. Matrice de modules (31) selon la revendication 1 ou 2, **caractérisée en ce que** les dispositifs de fixation (5, 6) sont réalisés sur les parois latérales du boîtier de module (2).

4. Matrice de modules (31) selon l'une des revendications précédentes, **caractérisée en ce qu'**au moins une des deux électrodes (16) du module générateur de plasma (1) respectif est logée au niveau d'une première extrémité d'un des deux éléments conducteurs de gaz, de sorte que l'au moins une des deux électrodes (16) peut être déplacée, conjointement avec la première extrémité de l'un des deux éléments conducteurs de gaz, par rapport à l'autre des deux électrodes (16) et de l'autre des deux éléments conducteurs de gaz, en direction de la distance entre les électrodes (18), ce qui permet d'ajuster différentes valeurs pour la distance entre les électrodes (18) pour la formation de formes géométriques différentes de la décharge rendue incomplète par voie diélectrique (27) à la pression atmosphérique.

5. Matrice de modules (31) selon la revendication 4, **caractérisée en ce qu'**un des deux éléments conducteurs de gaz est logé, avec sa deuxième extrémité, opposée à la première extrémité, dans un palier de pivotement monoaxial, sur une structure de base (15) du module générateur de plasma (1) respectif ou est logé de manière rigide au niveau de la structure de base (15), et constitue lui-même une articulation de corps solide monoaxiale, dans laquelle l'une des deux électrodes (16) peut être pivotée, avec la première extrémité d'un des deux éléments conducteurs de gaz, autour du palier de pivotement ou de l'articulation de corps solide, dans laquelle, en option, un canal d'alimentation en gaz, fixe par rapport à la structure de base (15) du module générateur de plasma (1) respectif débouche entre les deux éléments conducteurs de gaz.

6. Matrice de modules (31) selon l'une des revendications 4 et 5, **caractérisée en ce que** les deux électrodes (16) du module générateur de plasma (1) respectif sont logées respectivement au niveau d'une première extrémité d'un des deux éléments conducteurs de gaz, de sorte que les deux électrodes (16) peuvent être déplacées respectivement, conjointement avec la première extrémité de l'élément conducteur de gaz respectif, l'une par rapport à l'autre en direction de la distance entre les électrodes (18).

7. Matrice de modules (31) selon l'une des revendications 4 à 6, **caractérisée en ce que**, sur des arêtes latérales des éléments conducteurs de gaz, au niveau desquelles, respectivement une électrode mobile (16) du module générateur de plasma (1) respectif est logée, s'emboîtent deux curseurs (8) du module générateur de plasma (1) respectif, qui modifient, lors de leur coulissement le long d'un guidage (9) s'étendant transversalement par rapport aux électrodes (16), modifient la distance entre les électrodes (18) du module générateur de plasma (1) respectif.

8. Matrice de modules (31) selon l'une des revendications précédentes, **caractérisée en ce qu'**un diaphragme diélectrique (23) du module générateur de plasma (1) respectif peut passer d'une position inactive entre les éléments conducteurs de gaz à une position active entre les électrodes (16) du module générateur de plasma (1) respectif, et inversement.

9. Matrice de modules (31) selon la revendication 8, en référence à la revendication 7, **caractérisée en ce que** le diaphragme diélectrique du module générateur de plasma (1) respectif est couplé aux curseurs (8) du module générateur de plasma (1) respectif.

10. Matrice de modules (31) selon l'une des revendications précédentes, **caractérisée en ce que** la source de haute tension (24) fait partie du module générateur de plasma (1) respectif.

11. Matrice de modules (31) selon la revendication 10, en référence directe ou indirecte à la revendication 4, **caractérisée en ce que** la source de haute tension (24) du module générateur de plasma (1) respectif peut être commutée, pour la formation de différentes formes géométriques de décharges, entre différents modes de fonctionnement dans lesquels elle applique différentes hautes tensions alternatives aux électrodes (16) du module générateur de plasma (1) respectif et dans laquelle la source de haute tension (24) peut être raccordée à une source de basse tension externe exclusivement par l'intermédiaire de raccordements et de lignes de basse tension.

12. Utilisation d'une matrice de modules (31) selon l'une des revendications précédentes, **caractérisée en ce que**, au canal d'aspiration (12) du module générateur de plasma (1) respectif, est raccordé un ventilateur d'aspiration et **en ce que** le gaz de travail guidé par les éléments conducteurs de gaz (14) arrive entre les électrodes (16) du module générateur de plasma (1) respectif, traverse la distance entre les électrodes (18) et, de là, entre, autour de l'au moins une des deux électrodes (16), dans une embouchure en forme de fente (30) du canal d'aspiration (12), à travers lequel il est aspiré par le ventilateur d'aspiration.

13. Utilisation selon la revendication 12, **caractérisée en ce que** de l'air, en tant que gaz de travail, entre, à partir d'un environnement (11) du module générateur de plasma (1) respectif, à travers un canal d'alimentation en gaz (10), entre les éléments conducteurs de gaz (14).

14. Utilisation d'une matrice de modules (31) selon l'une des revendications 1 à 11 ou utilisation selon la revendication 12 ou 13, **caractérisée en ce que** différentes formes géométriques de décharge rendue incomplète par voie diélectrique (27) à la pression atmosphérique sont produites les unes après les autres dans le temps ou les unes à côté des autres dans l'espace, dans laquelle les différentes formes de décharges comprennent
- une décharge rendue incomplète par voie diélectrique (27) directe entre les deux électrodes (16) d'une part et un objet (25) disposé en face de celles-ci d'autre part,
- une décharge rendue incomplète par voie diélectrique (27) entre les deux électrodes (16), qui est soufflée sous la forme d'un jet de plasma (28), sur un objet (25) disposé en face de celles-ci et
- une décharge glissante coplanaire (29) entre une des deux électrodes (16) et un objet (25) disposé en face des électrodes (16), ainsi qu'entre l'objet (25) et l'autre des deux électrodes (16),
dans laquelle, pour la formation des différentes formes géométriques de décharges, différentes valeurs de la distance entre les électrodes (18) sont ajustées.

15. Utilisation selon la revendication 14, **caractérisée en ce que** les sources de haute tension (24) fonctionnent, pour les différentes formes de décharges, dans différents modes de fonctionnement, dans laquelle
- pour la décharge rendue incomplète par voie diélectrique (27) directe, une même tension alternative est appliquée au niveau des deux électrodes (16) par rapport à l'objet (25) ou à la terre et
- pour le jet de plasma (28) et la décharge glissante coplanaire (29), une tension alternative est appliquée entre les deux électrodes (16).
